Europäisches Patentamt

European Patent Office

Office européen des brevets

(11) Veröffentlichungsnummer: **0 279 288**
**A2**

(12) **EUROPÄISCHE PATENTANMELDUNG**

(21) Anmeldenummer: 88101594.5

(22) Anmeldetag: 04.02.88

(51) Int. Cl.⁴: **C07C 51/265** , C07C 63/04 , C07C 63/26 , C07C 67/03 , C07C 69/82 , B01J 19/26

(30) Priorität: 14.02.87 DE 3704720

(43) Veröffentlichungstag der Anmeldung:
24.08.88 Patentblatt 88/34

(84) Benannte Vertragsstaaten:
BE DE ES FR GB GR IT NL

(71) Anmelder: Hüls Troisdorf Aktiengesellschaft
Postfach 11 65
D-5210 Troisdorf(DE)

(72) Erfinder: Leuck, Hans
Robert-Koch-Strasse 4
D-5210 Troisdorf 15(DE)
Erfinder: Westermann, Hans-Jörg
Hitzbroicher Weg 18
D-5210 Troisdorf 15(DE)

(54) Verfaren und Vorrichtung zur Herstellung von Benzolcarbonsäuren bzw. Benzoldiarbonsäureestern.

(57) Es wird ein Verfahren sowie eine Vorrichtung zur Herstellung von Benzolcarbonsäuren bzw. Benzolcarbonsäureestrn oder deren Gemischen vorgeschlagen, wobei insbesondere gleichzeitig p-Toloylsäure aus p-Xylol und Terephthalsäuremonomethylester aus p-Toloylsäureester hergestellt wird, wobei die Oxidation durch Zugabe Sauerstoff enthaltender Gase und Oxidatorflüssigkeit in Wirbelstromdüsen unter Bildung einer hochdispersen Reaktionsphase erfolgt und die Wirbelstromdüsen in die Oxidatorflüssigkeit münden.

Figur 1

EP 0 279 288 A2

## Verfahren und Vorrichtung zur Herstellung von Benzolcarbonsäuren bzw. Benzoldicarbonsäureestern

Die Erfindung betrifft ein verbessertes Verfahren zur Herstellung von Benzolcarbonsäuren bzw. Benzoldicarbonsäureestern oder deren Gemischen durch Oxidation von Xylolen oder Toloylsäureestern mittels Sauerstoff oder Saurestoff enthaltenden Gasen in Gegenwart - schwermetallhaltiger Katalysatoren bei erhöhten Temperaturen und erhöhtem Druck.

Es ist bekannt, Terephthalsäure-dimethylester dadurch herzustellen, daß man p-Xylol (PX) mit Luft in flüssiger Phase oxidiert. Hierbei wird zunächst eine Methylgruppe des PX in Gegenwart von Schwermetallkatalysatoren unter Bildung von p-Toloylsäure oxidiert, diese dann mit Methanol zu p-Toloylsäuremethylester (PT-Ester = PTE) verestert, der anschließend gemeinsam mit PX zum Terephthalsäuremonomethylester weiter oxidiert und mit Methanol in Terephthalsäure-dimethylester überführt wird.

Beide Oxidationsvorgänge werden, soweit möglich, gemeinsam ausgeführt. Die vorgelegte flüssige Reaktionsphase wird vorzugsweise aus frischem PX und dem aus der Veresterung stammenden Betriebsester mit hohem Anteil PTE und Anteilen von Nebenprodukten hergestellt, wobei die Menge vone Bertriebsester überwiegt. Diese als Ausgangsmischung verwendete Reaktionsphase erlaubt den fließbaren Zustand aller zu handhabenden Stoffe während des Herstellverfahrens.

Der Fortschritt der Oxidation der Ausgansmischung zu der Oxidatorflüssigkeit mit Soll-Gehalten der Zielprodukte wird durch Messung der Säurezahl in mg KOH/g im "Oxidator" genannten Reaktor bestimmt. Die Selektivität der Oxidation wird durch Analyse des Abgases erfaßt, worin ein Gehalt von CO und $CO_2$ eine unerwünschte Dicarboxylierung und der Gehalt von Rest-$O_2$ die Ausnutzung des Sauerstoffs anzeigt.

Großtechnisch wird beim konventionellen Verfahren kontinuierlich mit einem Druck zwischen 0,5 und 1,1 MPa und Temperaturen von 135 bis 179°C gearbeitet. Man ist hierbei bestrebt, mit hohem Luftangebot bzw. hohen Raumzeitausbeuten bei möglichst weitgehendem Sauerstoffumsatz und geringer $CO/CO_2$-Abspaltung bzw. Nebenproduktbildung zu arbeiten und gleichzeitig in einem eng begrenzten, dem Reaktionsfortschritt angepaßten Temperaturbereich der Oxidationszone, die beträchtliche Reaktionswärme abzuführen. Die Oxidatoren sind z.B. als Blasensäulenreaktoren von 10 oder mehr m Höhe ausgebildet, in deren vertikaler Flüssigkeitssäule das Reaktionsgas mittels Gasverteilern eingeleitet wird.

Begrenzung sind durch Schaumbildung und $O_2$-Durchbrüche gegeben, die die Betriebssssicherheit und Wirtschaftlichkeit herabsetzen. Die Ableitung und Rückgewinnung der Reaktionswärme erfolgt durch verdampfendes Kesselspeisewasser in Wärmeaustauschsystemen inner-oder außerhalb des Oxidators. Standardausrüstung sind hochentwickelte, aber aufwendige Röhrenreaktoren.

Bei der anderen Verfahrensweise wird mit Gasstrahldüsen das Oxidationsgas in die Oxidatorflüssigketi eingeleitet, um hohe Stoffumsätze zu erzielen, wobei die Reaktionswärme wie oben beschrieben angeführt wird. Auch bei diesem Verfahrensweg ist die eingeleitete Luftmenge begrenzt durch erhöhte Sauerstoffanteile und Verbrennungsprodukte im Abgas sowie unvermediliche Schaumbildung im Oxidator. Es müssen sehr hohe bzw. sehr lange Oxidatoren verwendet werden.

Durch die start exotherme Oxidationsreaktion sind in beiden Verfahren örtliche Temperaturspitzen nicht vermeidbar, was eine erhöhte Bildung von CO und $CO_2$, niederen aliphatischen Säuren und hochmolekularen Oxidationsprodukten mit der Folge von Ausbeuteverlusten bewirkt.

Es bestand daher die Aufgabe, die Selektivität und Stoffausbeute der Reaktion, d.h. die Bildung von Terephthalsäuremonomethylester aus PTE und von p-Toloylsäure aus PX sowie nach Möglichkeit die Oxidationsgeschwindigkeit zu erhöhen und durch Reduzierung der Verweilzeit der flüssigen Produkte im Oxidator deren thermische Belastung zu senken und die dadurch entstehende erhöhte Wärmemenge pro Zeiteinheit bei optimalen Reaktionstemperaturen zu beherrschen.

Gegenstand der Erfindung ist das Verfahren nach Anspruch 1 und dessen Unteransprüchen sowie der Oxidator zur Durchführung des Verfahrens. Erfindungsgemäß wird zur Herstellung von Benzolcarbonsäure bzw. Benzoldicarbonsäureestern und deren Gemischen durch Oxidtion von Xylolen und/oder Toloylsäureestern als Ausgangsstoffe mittels Sauerstoff oder Sauerstoff enthaltenden Gasen in Gegenwart von - schwermetallhaltigen Oxidationskatalysatoren bei Temperaturen von 110 bis 200° C, vorzugsweise 130 bis 170° C, unter Anwendung von erhöhtem Druck von 0,2 bis 1,5, vorzugsweise 0,5 bis 0,9 MPa, die katalysatorhaltige Oxidatorflüssigkeit mit Hilfe einer Pumpe im Kreislauf geführt und unter Zusatz von Sauerstoff oder Sauerstoff enthaltenden Gasen mittels einer oder mehrerer Wirbelstromdüsen als hochdisperse Reaktionsphase zerstäubt zur Reaktion gebracht und in den Reaktor zurückgeführt wird.

Als Wirbelstromdüsen werden Zweistoffdüsen mit "innerer" Vermischung verstanden (vgl. ULLMANN, Band 2, Seite 256, Abb. 4 rechts) mit Zusammenführung von zwei Stoffen im Inneren der Düse und nur einer (engen) Austrittsöffnung, in deren Bereich der Gas-Flüssig-Mischpunkt mit der höchsten Massengeschwindigkeit liegt.

Das Gas wird in den inneren Mischraum parallel zu der Düsenachse oder vorzugsweise im Winkel zur Achse eingeführt. Auf die Austrittsöffnung der Düse kann ein Mischrohr (Diffusor) aufgesetzt sein.

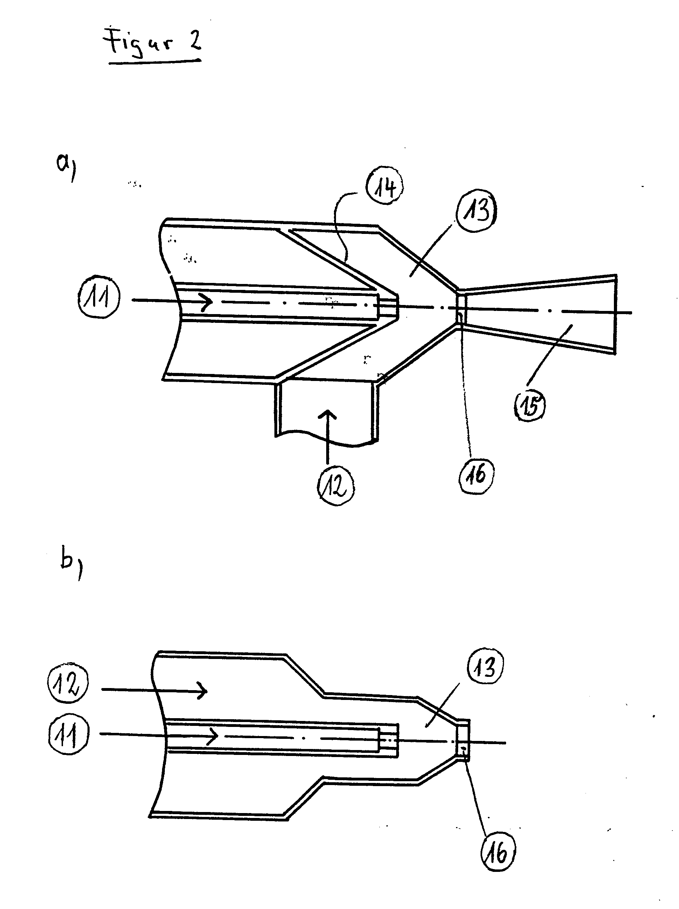

In Figur 2 sind Düsen des Typs Wirbelstromdüse mit innerer Vermischung in einer in der Düse liegenden Mischzone gezeigt. Gas 12 und Flüssigkeit, d. h. die von der Pumpe kommende Oxidatorflüssigkeit 11 mischen sich in der Düse in der inneren Mischzone 13 und treten vermischt aus der Düsenöffnung 16 aus. Gegebenenfalls trifft ein im Winkel zur Achse eintretendes Gas auf einen Drallkörper in Art eines Kegels 14 (vgl. Figur 2a). Wahlweise können Düsen gemäß Figure 2a oder 2b einen Düsenaufsatz bzw. Diffusor 15 aufweisen.

Im Gegensatz dazu stehen Düsen mit getrennten, z. B. konzentrischen Austrittsöffnungen aus der Düse für jeden Stoff und äußerer" Vermischung der Stoffe in einer (langen) Mischzone außerhalb der Düse, wobei Düsen mit "äußerer" Vermischung erfindungsgemäß nicht brauchbar sind, weil Gasdurchbrüche auftreten, sowie geringe Umsätze und die Bildung von Nebenprodukten bei der Reaktion erfolgen.

Es wurde nämlich gefunden, daß die genannten Nachteile der bekannten Verfahren wesentlich vermindert, die Wärmeabfuhr bei hohem Luftangebot und hohen Sauerstoffumsätzen bzw. hohen Raumzeitausbeuten besser beherrscht und gleichzeitig die Verbrennungsverluste bzw. die Bildung unerwünschter Nebenprodukte gesenkt werden, dadurch daß die Umsetzung der Ausgangsstoffe der flüssige Reaktionsphase bzw. der sich bildenden Oxidatorflüssigkeit durch Zusatz der Sauerstoff enthaltenden Gase in Wirbelstromdüsen vorgenommen wird, nachdem aus dem Oxidationsgas und der Oxidatorflüssigkeit eine hochdisperse Phase, die zur Reaktionsphase wird, erzeugt wurde. Überraschend hat sich gezeigt, daß im Inneren der Wirbelstromdüsen bzw. direkt dahinter eine sehr vollständige Umsetzung des Sauerstoffs erfolgt. Die eigentliche Reaktion findet auf sehr kleinem Raum bereits in der Wirbelstromdüse statt. Schon kurz hinter dem Austritt der Wirbelstromdüse wird in der Oxidatorflüssigkeit dieselbe geringe Menge Sauerstoff gefunden, die sich auch im abgetrennten Abgas vorfindet. Die umsetzbare, sauerstoffhaltige Gasmenge ist wesentlich, mindestens um das 4fache gegenüber bekannten Verfahren gesteigert. Es ist daher möglich, gegenüber bekannten Verfahren die Reaktionsgeschwindigkeit und damit die Raumzeitausbeute wesentlich zu steigern. Das Oxidatorvolumen bzw. die Höhe des Oxidators ist erheblich verkleinert bei gleichzeitiger Erhöhung des Stoffumsatzes, Erhöhung der Selektivität und Verminderung der Bildung von Nebenprodukten. Es gelang überraschend, die Temperaturführung der stark exothermen Reaktion besser zu beherrschen und wesentlich höhere Wärmemengen in der Zeiteinheit abzuführen. Die Funktion des Oxidators ist völlig verändert:

Der Reaktionsraum ist klein und besteht im wesentlichen nur noch aus einer oder mehreren ebenfalls kleinen Wirbelstromdüsen. Der Oxidator dient im wesentlichen nur noch als Pumpenvorlage und als Trennraum für die Abgase. Die Wirbelstromdüsen sollen erfindungsgemäß in die Oxidatorflüssigkeit münden. Jedoch ist hinter dem Austritt der Wirbelstromdüsen nur noch z.B. 0.3 bis 0.5 m Niveau Oxidatorflüssigkeit erforderlich. Sauerstoffdurchbrüche sind trotzdem vermieden.

Sehr bevorzugt ist vorgesehen, die Temperatur der eng begrenzten Reaktionszone auf exakte Soll-Werte einzustellen, dadurch, daß mittel einer Pumpe dem Oxidator entnommene Oxidatorflüssigkeit vor Eintritt in die Wirbelstromdüsen gekühlt wird, was mit einem Wärmeaustauscher im Kreislauf oder im Nebenschluß hierzu erfolgen kann. Weiterhin kann die Kreislaufflüssigkeit dadurch gekühlt werden, daß eine bei Reaktionstemperatur verdampfende inerte Flüssigkeit, vorzugsweise Wasser oder niedrig siedende organische Flüssigkeiten zugemischt werden. Die Zugabe der inerten Flüssigkeit erfolgt zweckmäßig in die Wirbelstromdüsen.

Vorzugsweise beträgt das Volumenverhältnis Gas zu Flüssigkeit unter Normbedingungen 1 bis 20 (d. h. 1 bis 20 Vol.-Teile Oxidationsgas zu einem Vol.-Teil Flüssigkeit), sehr bevorzugt 3 bis 8 (d. h. 3 bis 8 Vol.-Teile Gas zu einem Teil Oxidatorflüssigkeit aus der Pumpe). Der Düsenvordruck liegt höher als der Druck im Oxidator, vorzugsweise liegt der Düsenvordruck (der aus der Pumpe stammenden Oxidatorflüssigkeit) 0,1 bis 2,0 MPa, sehr bevorzugt 0,2 bis 1,0 MPa höher als der Druck im Oxidator.

Erfindungsgemäß kann in vorteilhafter Weise vorgesehen werden, mit dem Oxidationsfortschritt, d.h. mit steigender Säurezahl, den Düsenvordruck gegenüber dem Druck des Oxidators abzusenken oder zu erhöhen. In gleicher Weise kann vorgesehen werden, die Menge des sauerstoffhaltigen Gases zu der Menge der umgepumpten und den Wirbestromdüsen zugeführten Oxidatorflüssigkeit zu vermindern.

Entsprechend dem Reaktionsfortschritt kann die Temperatur im Oxidator verändert, vorzugsweise erhöht werden.

Es hat sich als zweckmäßig erwiesen, in den Wirbelstrom am Gas/Flüssig-Mischpunkt, d. h. am engsten Querschnitt der Zweiphasenströmung, Massengeschwindigkeiten von 1 500 bis 6 000 kg/sec m² einzuhalten, vorzugsweise sind Massengeschwindigkeiten von 2 000 bis 4 000 kg/sec m² vorgesehen.

In den Wirbelstromdüsen sind unter den genannten Bedingungen Potentialgeschwindigkeiten (U) von 15 vis 45 m/sec, vorzugsweise 20 bis 35 m/sec, zur Erzielung eines vorteilhaften Zerteilungsgrads der Flüssigphase vorgesehen.

Als Potentialgeschwindigkeit wird dabei der Wurzelwert der 2-fachen Druckdifferenz zwischen dem Düsenvordruck und dem Oxidatordruck, dividiert durch die Flüssigkeitsdichte, verstanden.

Es ist möglich, stehende oder liegende Reaktoren mit einer oder mehreren Wirbelstromdüsen auszurüsten. Als Wirbelstromdüsen werden dabei Düsen verstanden, in denen nach Umwandlung des Druckgefälles zwischen Düsenvor-und Oxidatordruck in Geschwindigkeitsenergie das Saurerstoff enthaltende Gas und Oxidatorflüssigkeit gemischt wird und dabei eine starke Verwirbelung von Gas und Flüssigkeit bewirkt. In dieser sich dabei ausbildenden hochdisperson Phase, die mit hoher Geschwindigkeit in die Reaktorflüssigkeit eintritt, erfolgt der größte Teil des Stoffumsatzes.

Art und Menge der Katalysatoren sind gegenüber dem konventionellen Verfahren unverändert. Im allgemeinen werden Kobaltsalze in Mengen von 50 bis 500 ppm Co, und bevorzugt zusätzlich Mangansalze in Mengen von 5 bis 50 ppm Mn, vorzugsweise als Acetate eingesetzt.

Für Versuchszwecke können Acetate in Lösungen niedriger Fettsäuren oder anderer Carbonsäuren verwendet werden. In der Technik können Acetate oder Salze aliphatischer Carbonsäuren ($C_2$ bis $C_{18}$) in Lösung dieser Carbonsäuren oder der Oxidatorflüssigkeit verwendet werden, wobei Cobalt plus Mangan oder Cobalt allein oder Mangan allein als Salz oder zusammen mit°Verbindungen bzw. Salzen von a. B. Mo oder Cr verwendet werden.

Die Oxidation wurde diskontinuierlich bis zu einer festgelegten Säurezahl durchgeführt und dann die Oxidatorflüssigkeit aufgearbeitet. Es zeigte sich, daß gegenüber dem Standardverfahren in Blasensäulenreaktoren bei gleicher Säurezahl, und somit gleichem Oxidationsgrad, die Selektivität der Reaktion bezüglich der Zielprodukte erheblich erhöht und somit die Menge der Nebenprodukte entsprechend erniedrigt war, was aus den während einer Oxidationscharge anfallenden, gegenüber der konventionellen Arbeitsweise verminderten $CO_2$-und $O_2$-Mengen hervorgeht. Überraschend war insbesondere eine um das mehrfache erhöhte durchsetzbare und nutzbare Luftmenge pro Volumen- bzw. Gewichtseinheit Oxidatorflüssigkeit mit entsprechend erhöter Raumzeitausbeute, d.h. wesentlich verkleinertem Apparatevolumen bei mindestens gleicher Produktionsmenge. Somit konnte, trotz vergleichsweise kleinem Oxidatorvolumne und gegenüber dem konventionellen Verfahren mehrfachem Wärmeanfall, die Temperatur in der Reaktionszone besser beherrscht, Luftdurchbrüche vermieden, zudem eine erhöhte Selektivität des Produkts und damit eine erhöhte Ausbeute - bezogen auf den Ausgangsstoff - erzielt werden.

Weiterer Gegenstand der Erfindung is die Vorrichtung zur Durchführung des Verfahrens, bestehend aus einem temperierbaren, unter Druck stehenden, liegenden oder stehenden, diskontinuierlich oder kontinuierlich betriebenen Oxidator mit Zuleitung der Ausgangsstoffe, Ableitung des Produkts sowie Gasableitung und einem Kreislauf der Oxidatorflüssigkeit, wobei der Kreislauf aus einem Ablauf aus dem Oxidator, einer den Druck erhöhenden Pumpeneinrichtung und einer oder mehrerer in den Oxidtor unter dem Flüssigkeitspegel angeordneten Wirbelstromdüsen mit Zuleitung von Oxidationsgas. Vorzugsweise ist der Oxidator mit Zuführung von verdampfbarer Flüssigkeit, vorzugsweise vor oder in die Wirbelstromdüsen und/oder einer Kühleinrichtung, vorzugsweise als Wämeaustauscher, zur Temperaturabsenkung der Kreislauffüssigkeit vor den Wirbelstromdüsen versehen.

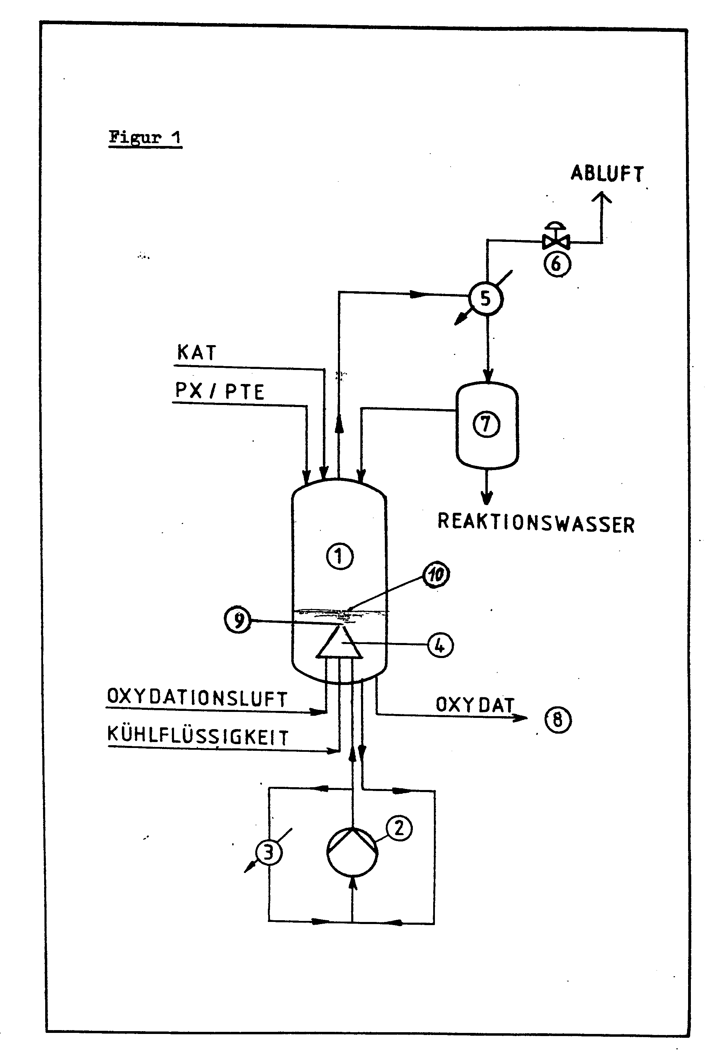

In der Zeichnung der Figur 1 ist die bevorzugte Ausführungsform der erfindungsgemäßen Apparatur schematisch dargestellt, wobei Wärmeaustauscher 3 oder die Zufuhr von verdampfender Kühlflüssigkeit wahlweise im Betrieb zuschaltbar sind.

Die im Oxidator 1 vorgelegten, katalysatorhaltigen Ausgansstoffe der flüssigen Reaktionsphase aus PX und Betriebsester werden mit der Kreislaufpumpe 2 nach Wärmeabfuhr und Temperatureinstellung im Wärmeaustauscher 3 unter Zumischung der Oxidationsluft in der Wirbelstromdüse 4 zerstäubt und zur Reaktion gebracht. Die weitgehend von Sauerstoff freie Gasphase verläßt über den Kondensator das System, wobei das Abgas über das Entspannungsventil 6 und eine nicht gezeichnete Abgasreinigung abgeführt wird.

Das Kondensat wird im Phasenabscheider 7 in eine ggf. aufzuarbeitende wäßrige Phase und in die zurück in den Oxidator geführte aromatische Phase getrennt wird. Die Oxidatorflüssigkeit mit einer festgelegten Säurezahl wird aus dem Oxidator über die Leitung 8 abgezogen. Under dem Flüssigkeitsspiegel 10 is das Wirbelstromdüsensystem 4 aus einer oder mehreren Düsen mit Zuführung für Oxidationsluft und ggf. vorgesehener Zuführung für verdmapfende inerte Flüssigkeiten als Kühlflüssigkeit sowie Zuführung von Kreislaufflüssigkeit, d.h. Oxidatorflüssigkeit, vorgesehen; aus der Austrittsöffnung 9 jeder Düse entweicht die hochdisperse Reaktionsphase in die aus der flüssigen Reaktionsphase entstehende Oxidatorflüssigkeit.

In den Beispielen beträgt die Massengeschwindigkeit 2 900 kg/sec m². Es wird eine Düse mit innerer Mischzone verwendet. Die Ausgangsstoffe werden mit einer Temperatur von 60 °C eingefüllt und mit dem als Erhitzer geschalteten Wärmeaustauscher 3 auf 130 °C erwärmt. Die Ausgangsstoffe mit Säurezahl 0 werden allgemein bis zu Säurezahlen von bis zu 300, vorzugsweise bis zu 250 oxidiert.

Beispiel 1

In einem Oxidationssystem nach dem Schema der Zeichnung mit einem Reaktor von 300 mm Durchmesser werden 13,5 kg p-Xylol (99,3 Gew.-%) und 31,5 kg Betriebsester mit 87 Gew.-% PTE vorgelegt mit Katalysatorgehalten von 150 Gew.-ppm Co und 13 Gew.-ppm Mn als Acetate. Nach Erreichen der Starttemperatur von 130° C wurden über 4,1 Stunden bei einem Reaktordruck von 0,8 MPa und einem Düsenvordruck von 1,2 MPa durchschnittlich Vn = 9,0 m³/h Luft in die Wirbelstromdüse eingeleitet und die Temperatur im Laufe der Reaktion auf 165° C gesteigert. Bei einer Säurezahl von 197 wurde die Oxidation abgebrochen. Die Temperatur im Kreislauf wurde gegenüber der Temperatur im Oxidator mit dem Wärmeaustauschr um 4 bis 10° C vermindert. Die insgesamt eingeleitete Luftmenge betrug Vn = 32,7 m³. Die laufend gemessene Abgaszusammensetzung hatte im Mittel die Werte von 1,45 Vol.% CO₂, 1,6 Vol.% O₂ sowie 1,15 Vol.% Sonstige. Daraus errechnet sich die Selektivität von 94,1 %, d.h. 1,85 % mehr als beim konventionellen Verfahren bei gleicher Säurezahl, was durch die hergestellten Mengen an Zielprodukten bestätigt wurde. Die Raumzeitausbeute, definiert als Koeffizient der eingesetzten Luft Vn/h, bezogen auf das Reaktionsvolumen, lag bei 180 bis 320 m³ Luft/hm³ in verschieden gleichartigen Durchgängen. Bei konventionellen Blasensäulenreaktoren liegt dieser Wert im Mittel bei 40 bis 45 m³Luft/hm³.

Beispiel 2

Entsprechend Beispiel 1 bezüglich Katalysator, Druck und Reaktionsgeschwindigkeit wurde die Oxidation mit einer insgesamt eingesetzten Luftmenge von Vn = 33,8 m³ bis zum Erreichen einer Säurezahl von 189 wiederholt. Die mittlere Abgaszusammensetzung betrug 1,38 Vol.% CO₂, 3,0 Vol.% O₂ und 1,12 Vol.% Sonstige. Die Selektivität betrug 93,5 %, d.h. 1,5 % mehr als bei gleicher Säurezahl in konventionellen Blasensäulenreaktoren.

**Ansprüche**

1. Verfahren zur Herstellung von Benzolcarbonsäuren bzw. Benzoldicarbonsäureestern und deren Gemischen durch Oxidation von Xylolen und/oder Toloylsäureestern als Ausgangsstoffe mittels Sauerstoff oder Sauerstoff enthaltenden Gasen in Gegenwart von schwermetallhaltigen Oxidationskatalysatoren bei Temperaturen von 110 bis 200° C, vorzugsweise 130 bis 170°C, unter Anwendung von erhöhtem Druck von 0,2 bis 1,5, vorzugsweise 0,5 bis 0,9 MPa, **dadurch gekennzeichnet,** daß die katalysatorhaltige Oxidatorflüssigkeit mit Hilfe einer Pumpe im Kreislauf geführt und unter Zusatz von Sauerstoff oder Sauerstoff enthaltenden Gasen mittels einer oder mehreren Wirbelstromdüsen als hochdisperse Reaktionsphase zerstäubt zur Reaktion gebracht und in den Reaktor zurückgeführt wird.

2. Verfahren nach Anspruch 1, dadurch gekennzeichnet, daß durch die Pumpe im Kreislauf ein erhöhter Druck gegenüber dem Druck im Reaktor aufrechterhalten wird.

3. Verfahren nach einem der Ansprüche 1 oder 2, dadurch gekennzeichnet, daß das Volumenverhältnis Gas zu Flüssigkeit unter Normbedingungen von 1 bis 20, vorzugsweise 3 bis 8, und die Differenz von Düsenvordruck zum Druck im Oxidator 0,1 bis 3,0 MPa, vorzugsweise 0,2 bis 1,0 MPa, beträgt.

4. Verfahren nach einem vorangehenden Ansprüche, dadurch gekennzeichnet, daß die Temperatur der der Düse zugeführten Oxidatorflüssigkeit im Kreislauf vor Eintritt in die Wirbelstromdüse mit Hilfe eines Wärmeaustauschers abgesenkt wird.

5. Verfahren nach einem der vorangehenden Ansprüche, dadurch gekennzeichnet, daß die der Düse zugeführte Oxidatorflüssigkeit mit einer bei Reaktionstemperatur verdampfenden inerten Flüssigkeit vermischt wird.

6. Verfahren nach einem der vorangehenden Ansprüche, dadurch gekennzeichnet, daß die Einstellung der Oxidationstemperatur in den Wirbelstromdüsen entsprechend dem Wärmeanfall bei der Reaktion durch Temperatursenkung in der Kreislaufflüssigkeit vor dem Eintritt in die Düsen und/oder durch dosierte Zugabe von verdampfenden inerten Flüssigkeiten erfolgt.

7. Verfahren nach einem der vorangehenden Ansprüche, dadurch gekennzeichnet, daß in den Wirbelstromdüsen am Gas/Flüssig-Mischpunkt Massengeschwindigkeiten von 1.500 bis 6.000, vorzugsweise 2.000 bis 4.000 kg/sec m² eingehalten werden.

8. Vorrichtung zur Durchführung des Verfahrens nach einem der vorangehenden Ansprüche, dadurch gekennzeichnet, daß ein temperierbarer, unter Druck stehender, leigender oder stehender, diskontinuierlich oder kontinuierlich betriebener Oxidator mit Zuleitung der Ausgangsstoffe, Ableitung des Produkts sowie Gasableitung und einem Kreislauf der Oxidatorflüssigkeit versehen ist, wobei der Kreislauf aus einem Ablauf aus dem Oxidator, einer den Druck erhöhenden Pumpeneinrichtung and einer oder mehrerer in den Oxidator unter dem Flüssigkeitsspiegel angeordneten Wirbelstromdüsen mit Zuleitung von Oxidationsgas und ggf. inerten verdampfbaren Flüssigkeiten besteht.

9. Oxidator nach Anspruch 8, versehen mit Zuführung von verdampfbarer Flüssigkeit, vorzugsweise vor oder in die Wirbelstromdüsen und/oder einer Kühleinrichtung, vorzugsweise als Wärmeaustauscher, zur Temperaturabsenkung der Kreislaufflüssigkeit vor den Wirbelstromdüsen.

10. Verfahren nach einem der Ansprüche 1 bis 7, dadurch gekennzeichnet, daß sich in der Düse Gas und Oxidatorflüssigkeit mischen und als Mischung durch die Düsenöffnung in die Oxidatorflüssigkeit im Oxidator austreten.

11. Vorrichtung nach einem der Ansprüche 8 oder 9, dadurch gekennzeichnet, daß sich in der Mischzone der Düse Gas und Oxidatorflüssigkeit mischen und als Mischung durch die Düsenöffnung in die Oxidatorflüssigkeit im Oxidator austreten.

**Figur 1**

ABLUFT

KAT

PX / PTE

REAKTIONSWASSER

OXYDATIONSLUFT

KÜHLFLÜSSIGKEIT

OXYDAT

Figur 2

a)

b)